# EUROPEAN PATENT APPLICATION

(11) **EP 3 939 600 A1**
(43) Date of publication of application: **19.01.2022**
(21) Application number: 20769730.1
(22) Date of filing: 12.03.2020
(51) Int. Cl.: A61K 35/16, A61K 35/18, A61K 38/18

(54) **METHOD FOR OBTAINING AND PRESERVING HIGH-PURITY GROWTH FACTORS AND USES THEREOF**

(30) Priority: 13.03.2019 ES 201930230
(71) Applicant: Active Bioregeneration Technologies, SL, S 28036 Madrid (ES)
(72) Inventor: SERRANO MORON, Juan Carlos, 28036 Madrid (ES)
(74) Representative: De Vidal Gonzalez, Maria Ester
(86) International application number: PCT/ES2020/070176
(87) International publication number: WO 2020/183051

(57) **Abstract**

The present invention refers to a method for obtaining and preserving high purity growth factors (with a concentration greater than 90%). The method comprises the steps of activating a plasma rich in growth factors, incubating, cooling, extracting growth factors with a concentration greater than 90% and deep-freezing. The growth factors obtained by the method of the present invention are used in tissue bioregeneration treatments.

## Description

### TECHNICAL FIELD

The present invention refers to a method for obtaining and preserving high purity growth factors (greater than 90%) applicable in clinical bioregeneration treatments, particularly in stomatology, odontology, aesthetics, traumatology, dermatology and rheumatology.

### BACKGROUND OF THE INVENTION

Platelets, commonly known for their role in the coagulation process, are cells that do not have a nucleus and therefore lack cell division properties. These cytoplasmic fragments from the megakaryocyte division contain three types of granules inside them: dense granules, lysosomes and alpha granules. Alpha granules are of great interest since, inside them, they house the so-called growth factors (GF) which have several tissue repair functions and marked angiogenic activity. There are different types of GFs, such as the beta transforming GF, epidermal or epithelial GF, vascular endothelial GF (angiogenic), acidic and basic fibroblast GF, and insulin-like IGF-I, among others.

GFs released by alpha granules have been shown to be effective in inducing tissue and functional regeneration in tissues with affected or underactive cells. For example, if a fibroblast in the skin has stopped producing collagen, an injection of autologous GFs in the area to be treated provides an inducing factor on the fibroblast (or target cell), resulting in the "new" production of collagen from the affected fibroblast.

Based on the above, the activated plasma rich in growth factors (PRGF) is of great scientific interest. Its obtaining method goes beyond carrying out a blood extraction and centrifuging it to separate the fraction richest in platelets. Platelets alone do not have regenerative action, but must be activated in order for alpha granules to release GFs.

Methods for obtaining platelet-rich plasma (PRP) are widely described in the state of the art. For example, ES2567603T3 discloses a method that allows generating a platelet-rich plasma concentrate, and comprises separating from a sample of whole blood mixed with anticoagulants a fraction of platelet-poor plasma and another fraction of platelet-rich plasma fraction by means of centrifugation. The method further includes a platelet count determination of platelet concentration after separation, and a method comprising mixing platelet-rich volumes with platelet-poor volumes so that a desired minimum concentration of 800,000 to 2,000,000 platelets/µL in all fractions is achieved.

Also, WO2014126931A describes a method for obtaining PRP for cosmetic (aesthetic) or therapeutic uses in the field of traumatology and rheumatology. Such method comprises the use of calcium chloride as an activator agent and inducer of gel formation from PRP. Furthermore, reference is made to the possibility of cryopreserving the material obtained at temperatures below -20°C by known cell preservation methods.

On the other hand, some PRP activation methods and the biological and medical utilities of the product are also described. Examples thereof can be found in ES2527967A2, which indicates the use of sodium gluconate as an activator of platelet-rich plasma.

In particular, moreover, a publication entitled "Platelet-rich plasma" (available from: https://issuu.com/robertohdzvides/docs/plasma rico en plaquetas.pptx) describes a method for obtaining PRP and its activation for application in odontology. This document describes the steps of extraction of the blood sample and deposit in containers, addition of 3.2% sodium citrate as anticoagulant, centrifugation and addition of 10% calcium chloride or calcium gluconate as platelet activator in order to obtain growth factors to be used in odontology.

Although several methods are described for obtaining plasma with growth factors, the vast majority of these methods achieve a plasma with a medium content of growth factors that must be used immediately since, otherwise, the quality of the plasma decreases in terms of the content of activated growth factors.

This is how the inventors of the present invention, after extensive and exhaustive experiments, have developed a method for obtaining pure growth factors and preserving them in such a way that they can be used later in an effective manner.

### DESCRIPTION OF THE INVENTION

Therefore, in a first aspect, the present invention refers to a method for obtaining and preserving high purity growth factors (above 90%) that can be used in cellular bioregeneration processes.

The method of the present invention consists in that, once a platelet-rich plasma is available, the following steps are carried out:
a) adding a platelet membrane breaking agent to a platelet-rich plasma in order to obtain a plasma rich in growth factors;
b) incubating the plasma rich in growth factors from step a) for 30 to 40 minutes at a temperature between 37 and 40°C;
c) cooling the plasma rich in growth factors from step b) for 5 to 7 minutes in order to obtain a gel and a supernatant, said supernatant containing growth factors at a concentration greater than 90%;
d) extracting the supernatant with growth factors from step c);
e) deep-freezing the supernatant with growth factors from step d) at a temperature between -40°C and -18°C.

The supernatant obtained in step c) contains no platelet residues.

The method of the present invention is preferably carried out in a sterile environment, optionally in a laminar flow hood.

In step a), the platelet membrane breaking agent is responsible for releasing the growth factors, optionally the platelet membrane breaking agent is sodium gluconate in solution, preferably with a concentration between 100 mg/mL. Preferably, the solution is added to the platelet-rich plasma at 20% v/v.

The growth factors (without platelet residues) obtained by the method of the present invention have been shown to be more effective when used in bioregeneration processes compared to the performance of other forms of plasma derivatives such as platelet-rich plasma, PRGF and activated PRGF. By using the growth factors of the present invention, a greater effect is achieved with lower doses of product and fewer applications, which shows a better assimilation by the patient.

Furthermore, the product is stored for up to 18 months and shows to maintain its effectiveness in terms of growth factor content. The product does not show loss of effectiveness when thawed up to three times.

### EXAMPLES

For a better understanding and in a non-limiting manner, the present invention is described in more detail in the embodiments and tests described below.

### Example 1. Obtaining and preserving pure growth factors

Inside a laminar flow hood, in a 6 milliliter Vacutainer tube, 1 milliliter of PRP was deposited with 0.2 milliliters of sodium gluconate in solution (with a concentration of 100 mg/mL).

The tube was incubated for 30 minutes at 40°C. Then, it was fan cooled for 5 minutes. This process was carried out in an ISM Equipment.

A gel and a supernatant with the growth factors were obtained. The tube was inverted and with the aid of a syringe the supernatant was withdrawn until the gel was very reduced.

Since the supernatant containing the pure growth factors loses its activity very quickly at room temperature, the material is deposited quickly (in a time significantly less than 5 minutes) in sterile vials.

Before being used, the vials are subjected to a bacteriological control to guarantee their sterility.

The sterile vials are then frozen at -18°C and, in some cases, at -40°C using dry ice.

## Claims

1. Method for obtaining pure growth factors, **characterized by** comprising the steps of:
a. adding a platelet membrane breaking agent to a platelet-rich plasma, in order to obtain a plasma rich in growth factors;
b. incubating said plasma rich in growth factors of step a) for 30 to 40 minutes at a temperature between 37 and 40°C;
c. cooling the plasma rich in growth factors of step b) for 5 to 7 minutes in order to obtain a gel and a supernatant, said supernatant containing growth factors at a concentration greater than 90%;
d. extracting the supernatant with growth factors from step c);
e. deep-freezing the supernatant with growth factors from step d) at a temperature between -40°C and -18°C.

2. The method of claim 1, **characterized in that** the platelet membrane breaking agent is sodium gluconate in solution.

3. The method of claim 2, **characterized in that** the sodium gluconate in solution has a concentration of 100 mg/mL and is added to the platelet-rich plasma at 20% v/v.

4. The method of any of the preceding claims, **characterized in that** it is carried out in a sterile environment.
